# EUROPEAN PATENT SPECIFICATION

(11) **EP 4 610 253 B1**
(45) Date of publication and mention of the grant of the patent: **09.09.2026**
(21) Application number: 23882716.6
(22) Date of filing: 26.10.2023
(51) Int. Cl.: C07D 213/77

(54) **CRYSTALS OF A NAPHTHALENE DERIVATIVE**
KRISTALLE EINES NAPHTHALINDERIVATS
CRISTAUX D'UN DÉRIVÉ DE NAPHTALÈNE

(30) Priority: 27.10.2022 JP 2022172131
(43) Date of publication of application: 03.09.2025
(73) Proprietor: Kyoto Drug Discovery & Development Co., Ltd., Ibaraki-shi, Osaka, 567-0085 (JP)
(72) Inventor: MUSASHI, Kunihiro, Ibaraki-shi, Osaka 567-0085 (JP); OKAMOTO, Masaki, Ibaraki-shi, Osaka 567-0085 (JP)
(74) Representative: Hoffmann Eitle
(86) International application number: PCT/JP2023/038651
(87) International publication number: WO 2024/090512

(56) References cited:
- EP-A1- 2 599 771
- WO-A1-2012/014994
- WO-A1-2012/043891
- WO-A1-2014/129495
- WO-A1-2015/033981
- WO-A1-2015/129809
- KAZUHIDE ASHIZAWA: "Iyakuhin Kessho no Bunshi Jotai ni Kansuru Bussei Hyoka (12) Shio Kesshokei no Saitekika to Kesshoka Gijutsu [Physico-Chemical Studies on the Molecular Details of Drug Crystals]", PHARM TECH JAPAN, JIHO, INC., JP, vol. 18, no. 10, 1 January 2002 (2002-01-01), JP , pages 81 (1629) - 96 (1644), XP008177312, ISSN: 0910-4739
- NORIYUKI TAKATA: "Soyaku Dankai ni Okeru Gen'yaku Form Screening to Sentaku - API form screening and selection in drug discovery stage", PHARM STAGE, TECHNICAL INFORMATION INSTITUTE, JP, vol. 6, no. 10, 1 January 2007 (2007-01-01), JP , pages 20 - 25, XP008145548, ISSN: 1346-3918

## Description

### TECHNICAL FIELD

The present invention relates to a crystal of KUS121 useful as a medicament, and a process for preparing the crystal.

### BACKGROUND OF THE INVENTION

Sodium 4-amino-3-[6-(4-fluoro-2-methylphenyl)pyridin-3-ylazo]naphthalene-1-sulfonate (hereinafter, also referred to as "KUS121") is a compound having a naphthalene structure, which has been now developed as a medicament for treating central retinal artery occlusion (Patent literature 1). KUS121 is dissolved to be locally administered as an injection. For a drug substance for manufacture before it is formulated, generally, it is stored as a crystalline drug substance that is generally considered to have excellent storage stability, and controlled about quality. It is because the drug substance needs to gain its high quality at this stage and to be stored for a certain period.

However, the storage stability of KUS121 crystals is not always high, for example, under normal accelerated test conditions, there is a marked tendency for crystallinity to decrease, crystal morphology to collapse, and water content to increase. Thus, from the viewpoint of quality control of pharmaceuticals, it has been necessary to store the crystals under severer storage conditions than usual. In addition, plural crystal forms have been identified as KUS121 drug substance, and thus it has been necessary to prepare a single crystal form having ideal stability, and also fix such stable process.

As mentioned above, it has been desired to find a more stable crystal of KUS121 drug substance, and also establish a process that can stably prepare such stable single crystal form with good reproducibility.

EP 2599771A1 describes a naphthalene derivative for use in treating VCP-mediated diseases.

### PRIOR ART

### (Patent Reference)

[Patent literature 1] WO 2012/014994
[Patent literature 2] EP 2599771A1

### SUMMARY OF INVENTION

### (Technical Problem)

The purpose of the present invention is to find a stable crystal of KUS121 as an industrial drug substance, and establish a process thereof.

### (Solution to Problem)

The present inventors started first the study about crystal forms of KUS121 to solve the above-mentioned problem, and found that there are as many as 20 types of crystal polymorphisms among those studies alone. The inventors further proceeded in the analyses and then found that these crystal polymorphisms include some solvates which contain solvents used in crystalizing processes, and also include some types of hydrates. After further study, the inventors have finally found that one of crystalline forms of KUS121 dihydrate has particularly excellent storage stability, and have established a process for specifically preparing only the dihydrate crystalline form. Based upon the new findings, the present invention has been completed.

The present invention includes the following embodiments.

### (Item 1)

A crystal of sodium 4-amino-3-[6-(4-fluoro-2-methylphenyl)pyridin-3-ylazo]naphthalene-1-sulfonate dihydrate, characterized by an X-ray powder diffraction pattern having at least four diffraction angle (2θ° ± 0.5°) peaks selected from 6.9°, 13.3°, 14.2°, 15.0°, 17.1°, 18.5°, 19.6°, 21.1°, 22.9°, 23.7°, 26.7°, 27.4°, 29.5°, 34.1°, and 36.1°.

### (Item 2)

A crystal of sodium 4-amino-3-[6-(4-fluoro-2-methylphenyl)pyridin-3-ylazo]naphthalene-1-sulfonate dihydrate according to item 1, characterized by an X-ray powder diffraction pattern having at least four diffraction angle (2θ° ± 0.2°) peaks selected from 6.9°, 13.3°, 14.2°, 15.0°, 17.1°, 18.5°, 19.6°, 21.1°, 22.9°, 23.7°, 26.7°, 27.4°, 29.5°, 34.1°, and 36.1°.

### (Item 3)

The crystal of Item 1 or 2, wherein the selected diffraction angle peaks are at least five peaks.

### (Item 4)

The crystal of Item 1 or 2, wherein the selected diffraction angle peaks are at least 10 peaks.

### (Item 5)

The crystal of Item 3, wherein the selected diffraction angle peaks are 6.9°, 13.3°, 14.2°, 15.0°, and 17.1°.

### (Item 6)

The crystal of Item 4, wherein the selected diffraction angle peaks are 6.9°, 13.3°, 14.2°, 15.0°, 17.1°, 19.6°, 21.1°, 26.7°, 27.4°, and 29.5°.

### (Item 7)

A process for preparing a crystal of sodium 4-amino-3-[6-(4-fluoro-2-methylphenyl)pyridin-3-ylazo]naphthalene-1-sulfonate dihydrate, comprising
(i) dissolving KUS121 in a water-containing solvent with appropriate heating, said solvent is a solvent commonly used in the manufacture of pharmaceuticals, wherein the solvent commonly used is an alcoholic solvent, and stirring the solution at 40°C to 100°C,
(ii) gradually cooling the solution to precipitate a crystal, and collecting the precipitated crystal on a filter, and
(iii) drying the crystal at a temperature of 30°C to 70°C in an atmosphere with relative humidity of 10% RH to 100% RH to give a crystal of sodium 4-amino-3-[6-(4-fluoro-2-methylphenyl)pyridin-3-ylazo]naphthalene-1-sulfonate dihydrate.

### (Item 8)

A process for preparing a crystal of sodium 4-amino-3-[6-(4-fluoro-2-methylphenyl)pyridin-3-ylazo]naphthalene-1-sulfonate dihydrate according to item 7, comprising
(i) dissolving KUS121 in a water-containing solvent at a concentration of 1%(w/v) to 20%(w/v) with appropriate heating, said solvent is a solvent commonly used in the manufacture of pharmaceuticals, wherein the solvent commonly used is an alcoholic solvent, and stirring the solution at 40°C to 100°C for 5 min or more,
(ii) cooling the solution to -20°C to 30°C at a cooling rate of 0.01°C/min to 1°C/min, and collecting the precipitated crystal on a filter, and
(iii) drying the crystal at a temperature of 30°C to 70°C in an atmosphere with relative humidity of 10% RH to 100% RH to give a crystal of sodium 4-amino-3-[6-(4-fluoro-2-methylphenyl)pyridin-3-ylazo]naphthalene-1-sulfonate dihydrate.

### (Item 9)

The process of Item 7 or 8, wherein the alcoholic solvent is methanol, ethanol, 1-propanol, and/or 2-propanol.

### (Item 10)

The process of any one of Items 7 to 9, wherein the drying step in (iii) is done under ambient pressure for 10 hours or more.

### (Item 11)

The process of any one of Items 7 to 9, wherein the drying step in (iii) is done at a temperature of 30°C to 50°C and a pressure of 2 kPa to 5 kPa for 10 hours or more in a drying chamber in which a tray filled with water is placed.

### (Item 12)

The process of any one of Items 7 to 9, wherein the drying step in (iii) is done at a temperature of 30°C to 50°C and a pressure of 1 kPa to 7 kPa for 5 hours or more in a drying chamber, while blowing nitrogen saturated with water vapor into the dry chamber.

### (Effect of the Invention)

The present crystal of KUS121 dihydrate is a crystalline form that has excellent storage stability among many other crystalline polymorphisms of KUS121, and is useful as a stable drug substance in manufacturing. In addition, the process for preparing the crystal of KUS121 dihydrate established in the present invention makes it possible to stably prepare the crystal of KUS121 dihydrate.

### BRIEF DESCRIPTION OF DRAWINGS

Fig. 1 shows an X-ray powder diffraction chart of the crystal of KUS121 dihydrate after drying in Example.
Fig. 2 shows the results of differential scanning calorimetry (DSC) and thermogravimetric analysis (TGA) of the crystal of KUS121 dihydrate after drying in Example.
Fig. 3 shows a Raman scattering spectrum (FT-Raman) of the crystal of KUS121 dihydrate after drying in Example.
Fig. 4 shows a photomicrograph (PLM) of the crystal of KUS121 dihydrate after drying in Example.
Fig. 5 shows an X-ray powder diffraction chart of the crystal of KUS121 anhydride of Example.
Fig. 6 shows an X-ray powder diffraction chart of the crystal of KUS121 monohydrate of Example.
Fig. 7 shows the results of differential scanning calorimetry (DSC) and thermogravimetric analysis (TGA) of the crystal of KUS121 monohydrate of Example.
Fig. 8 shows an X-ray powder diffraction chart of the crystal of KUS121 tetrahydrate of Example.
Fig. 9 shows the results of differential scanning calorimetry (DSC) and thermogravimetric analysis (TGA) of the crystal of KUS121 tetrahydrate of Example.

### DESCRIPTION OF EMBODIMENTS

The present compound, sodium 4-amino-3-[6-(4-fluoro-2-methylphenyl)pyridin-3-ylazo]naphthalene-1-sulfonate (KUS121), has the structure shown below, and the manufacturing process and spectral data thereof are disclosed in Patent literature 1.

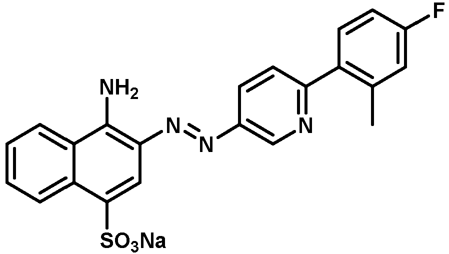

In the present invention, crystalline substances can be analyzed using conventional techniques such as X-ray powder diffraction (XRPD), differential scanning calorimetry (DSC), thermogravimetric analysis (TGA), Raman scattering spectrum (FT-Raman), and photomicrograph (PLM).

The 2θ values of X-ray powder diffraction patterns can vary slightly from instrument to instrument or sample to sample, thus the numbers stated herein are not absolute. In general, the measurement error of diffraction angles in an X-ray powder diffraction spectrum is, for example, about ± 0.2° in 2θ, and such degree of measurement error should be taken into consideration when analyzing X-ray powder diffraction data. Additionally, in the present invention, the value of the diffraction angle of the crystal of KUS121 dihydrate varies greatly between measuring instruments, thus the measurement error of the diffraction angle is even larger, for example, about ± 0.5° in 2θ should be taken into consideration. Furthermore, the intensity can vary depending on experimental conditions and sample preparation (preferred orientation). In the present invention, the values obtained by measurement using copper radiation (Cu Kα, 45 kV/20 mA, A=1.5418 Å) are shown.

### Preparation of crystal of KUS121 dihydrate

The process for preparing a crystal of sodium 4-amino-3-[6-(4-fluoro-2-methylphenyl)pyridin-3-ylazo]naphthalene-1-sulfonate dihydrate is characterized by comprising (i) dissolving KUS121 in a water-containing solvent with appropriate heating, said solvent is a solvent commonly used in the manufacture of pharmaceuticals, and stirring the solution with heating, (ii) cooling the heated solution to precipitate a crystal, and collecting the wet crystal on a filter, and (iii) drying the wet crystal.

Preferably, by adding a seed crystal in Step (ii), the reliability of controlling the formation of the crystal of KUS121 dihydrate can be improved.

The "solvent is a solvent commonly used in the manufacture of pharmaceuticals" defined in the above step (i) is not particularly limited as long as it is a solvent commonly used in the manufacture of pharmaceuticals, which includes, for example, an alcoholic solvent such as methanol, ethanol, 1-propanol, 2-propanol, 1-butanol, 2-butanol, 2-methoxyethanol, 1-methoxy-2-propanol, 2-methyl-1-propanol, 3-methyl-1-butanol, and 1-pentanol; and other solvents commonly used in pharmaceutical manufacturing, such as acetone, anisole, methyl acetate, ethyl acetate, propyl acetate, isopropyl acetate, n-butyl acetate, isobutyl acetate, t-butyl methyl ether, dimethylsulfoxide, diethyl ether, ethyl formate, heptane, methyl ethyl ketone, methyl isobutyl ketone, pentane, nitromethane, acetonitrile, acetone, 4-methoxy-2-pentanone, 1,2-dimethoxyethane, toluene, pyridine, tetrahydrofuran, cyclohexane, heptane, and bis(2-methoxyethyl) ether. In addition, it includes a mixed solvent of the above-mentioned solvents or a mixed solvents of these solvents and other solvents. It includes, preferably, methanol, ethanol, and 2-propanol, and more preferably ethanol.

The water content of the "water-containing solvent which is commonly used in the manufacture of pharmaceuticals" depends on the type of solvent used, but it is usually 5% to 90%; and preferably 10 to 50 % for methanol, 10% to 90% (more preferably 10% to 60%) for ethanol, and about 30% for 2-propanol.

The concentration of KUS121 dissolved in a water-containing solvent in the above step (i) may be 1%(w/v) to 20%(w/v), preferably 2%(w/v) to 10%(w/v).

The heating temperature in the above step (i) depends on the type of solvent used, but it may be 40 to 100°C, preferably 50 to 80°C, more preferably 60 to 80°C.
The stirring time in the above step (i) is not particularly limited as long as KUS121 is sufficiently dissolved, it is usually 5 minutes or more, preferably 10 minutes or more.

The cooling rate in the above step (ii) is usually 0.01 to 1°C/min, preferably 0.05 to 0.5°C/min. And, the cooling is carried out usually to -20 to 30°C, preferably to 0 to 30°C. After these temperatures are reached, usually the stirring is continued until the crystal is aged, or the stirring is stopped and allowed to stand.

As for the drying condition in the above step (iii), the drying is carried out usually at a temperature of 30°C to 70°C in an atmosphere of relative humidity 10% RH to 100% RH, preferably at a temperature of 30°C to 60°C in an atmosphere of relative humidity 30% RH to 95% RH, more preferably at a temperature of 40°C to 60°C in an atmosphere of relative humidity 40% RH to 70% RH. The drying time is usually 3 hours or more, preferably 10 hours or more.
And, the drying may be carried with blowing air as appropriate.

In addition, the drying may be carried out under reduced pressure as appropriate. The pressure under reduced pressure is usually 0.05 kPa to 20 kPa, preferably 1 kPa to 20 kPa. The temperature under reduced pressure is usually 30°C to 60°C, preferably 30°C to 50°C, and the humidity can be controlled by placing a tray filled with water in the drying chamber or by blowing nitrogen gas containing water vapor. A preferred embodiment under reduced pressure is drying at 30°C to 50°C, and at a pressure of 1 kPa to 7 kPa for 5 hours or more while blowing nitrogen saturated with water vapor into the drying chamber. Another preferred embodiment under reduced pressure is drying at 30°C to 50°C, and at a pressure of 2 kPa to 5 kPa for 10 hours or more in the drying chamber in which a tray filled with water is placed.

### EXAMPLES

The present invention is explained in further detail below through the following Examples, but these do not limit the present invention.

### (1) Analysis method

Each of the prepared crystals was analyzed using the following analysis methods under the conditions described therein.

### X-ray powder diffraction (XRPD)

### (Measurement condition 1)

X-ray powder diffraction data were acquired using PhotonMax high-flux 9 kW rotating cathode X-ray generator (SmartLab 9 kW, Rigaku). All diffractograms were acquired using copper radiation (CuKα, 45 kV/200 mA, λ = 1.5418 Å) and using Kβ filter to reduce unneeded radiation. The measurement was carried out by placing the sample gently crushed in an agate mortar on a measurement stand and then scanning the sample under 2θ scanning conditions with a scanning range of 3° to 40°, a scanning speed of 20°/min, and a step size of 0.02°.

### (Measurement condition 2)

X-ray powder diffraction data were acquired on Si zero-background wafers using a diffractometer (X'PertPro, PANalytical). All diffractograms were acquired using copper radiation (CuKα, 45 kV/40 mA, λ = 1.5418 Å) and X'celerator^{™} RTMS (Real Time Multi-Strip) detector and using a nickel filter to reduce unneeded radiation. Scanning was carried out in 2θ with a scanning range of 2° to 40° and a step size of 0.02°. The configuration on the input beam side was composed of a fixed diverging slit (1/4°), a 0.04 radian soller slit, an anti-scatter slit (1/4°), and a 10 mm beam mask. The configuration on the diffracted beam side was composed of a fixed divergent slit (1/4°) and a 0.04 radian soller slit.

### (Measurement condition 3)

X-ray powder diffraction data were acquired using a diffractometer (MiniFlex 600, Rigaku). All diffractograms were acquired using copper radiation (CuKα, 40 kV/15 mA, λ = 1.5418 Å) and using Kβ filter to reduce unneeded radiation. Scanning was carried out in 2θ with a scanning range of 5° to 50°, a scanning speed of 5°/min, and a step size of 0.02°.

### Differential scanning calorimetry (DSC)

Differential scanning calorimetry was performed using a differential scanning calorimeter (TA Instruments Q100 or Q2000, TA Instruments) equipped with an autosampler and a refrigeration cooling system. DSC thermograms were acquired by placing about 1 mg to 10 mg of the solid sample into a crimped aluminum pan and heating it at 10°C/min or 15°C/min under a 40 mL/min N₂ purge.

### Thermogravimetric analysis (TGA)

Thermogravimetric analysis was performed using thermogravimetric analyzer (TA Instruments Q50 or Q500, TA Instruments). TGA thermograms were acquired by placing about 1 mg to 10 mg of the solid sample into an aluminum pan and heating it at 10°C/min or 15°C/min under a 40 mL/min N₂ purge.

### Raman scattering spectrum (FT-Raman)

Raman spectra were measured using a Raman apparatus (Nicolet NXR9650 or NXR960 spectrometer, Thermo Electron) equipped with a 1064 nm Nd: a YVO4 excitation laser, an InGaAs and liquid nitrogen cooled Ge detector, and a MicroStage. All spectra were acquired in a condition of 4 cm-1 resolution and 64 to 1024 scans using the Happ-Genzel apodization function and two levels of zero filling. A neutral density filter (1.0 optical density) was used if detector saturation was observed.

### Photomicrograph (PLM)

Photomicrographs were taken using a BX60 polarizing microscope (Olympus) equipped with an Olympus DP70 camera.

### Moisture measurement

The water content of each crystal was measured using one of the following three methods.

### (Moisture measurement of crystal of KUS121 anhydride)

The water content was measured using a coulometric moisture meter (CA-100, Nitto Seiko Analytech), using about 0.5 g of the solid sample, Aquamicron GEX as the solvent, and 3 mg of Aquamicron SS as a titrant.

### (Moisture measurement of crystal of KUS121 dihydrate)

The water content was measured using a Karl Fischer moisture meter (AQUACOUNTER AQV-2100, HIRANUMA) equipped with an electrolytic bath stirrer for iodine generation and a constant current potentiometer, using about 0.2 g of the solid sample, Aquamicron GEX as the solvent, and 3 mg of Aquamicron SS-Z as a titrant.

### (Moisture measurement of crystals of KUS121 monohydrate and tetrahydrate)

The water content was calculated from DSC thermogram measured using differential scanning calorimetry (differential scanning calorimeter TA Instruments Q100 or Q2000, TA Instruments) and TGA thermogram measured using thermogravimetric analysis (thermogravimetric analyzer TA Instruments Q50 or Q500, TA Instruments). The measurement was carried out by placing about 1 mg to 10 mg of the solid sample in an aluminum pan and heating it at 10°C/min or 15°C/min under a 40 mL/min N₂ purge.

### (2) Preparation of crystal

The crystals of KUS121 dihydrate, anhydride, monohydrate, and tetrahydrate were prepared according to the following procedures. And, the drying process for the dihydrate crystal was carried out in two ways: under normal pressure and under reduced pressure.

### Preparation of dihydrate crystal (crystallization)

TO KUS121 (5.00 g) was added 90 % aqueous ethanol (120 mL), and the mixture was stirred at 67°C or higher to be dissolved. The solution was filtered while hot, and the vessel and filter were washed with 90 % aqueous ethanol (5 mL) and the washing solution was also filtered. The filtrate was heated to 67°C or higher, stirred for 30 minutes, and cooled to 60°C at a cooling rate of 0.1°C/min. The cooled solution was stirred at 60°C for one hour to age a crystal, and cooled to 22°C at a cooling rate of 0.05 to 0.5°C/min. The precipitated crystal was collected on a filter and washed with 90 % aqueous ethanol (5 mL) to obtain a wet crystal of KUS121 dihydrate (net yield: 97.3 %).

### Water content: 13.0 %

### Preparation of dihydrate crystal (normal pressure drying)

The wet crystal of KUS121 dihydrate (24.0 g, net 17.4 g) prepared through the above procedure was dried with blowing air at 50°C to obtain a dry crystal of KUS121 dihydrate (yield: 91.7 %).

### Water content: 7.4%

### Preparation of dihydrate crystal (vacuum drying)

The wet crystal (2.32 kg) of KUS121 dihydrate prepared through the above procedure was spread over two vats, and the vats were placed on a shelf in a vacuum dryer together with a beaker containing distilled water (4.56 g). Drying was carried out under reduced pressure for about 16 hours while controlling the external temperature of the dryer to 35°C and the degree of vacuum to 2.86 to 2.96 kPa to obtain 1.92 kg of a dry crystal of KUS121 dihydrate.

### Water content: 7.7%

### Analysis results of dihydrate crystal after drying

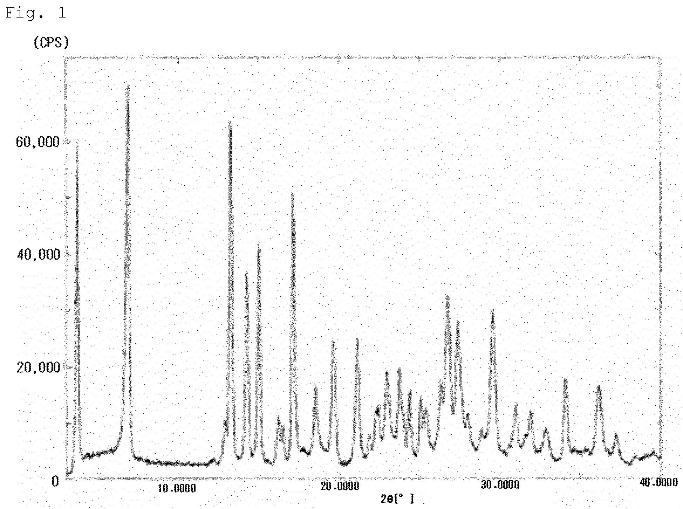

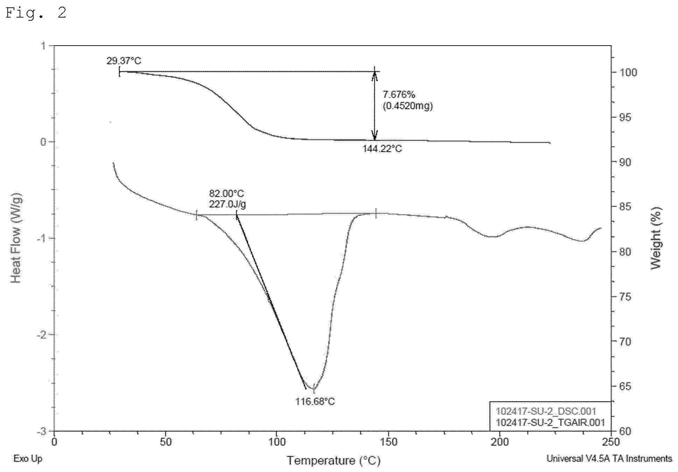

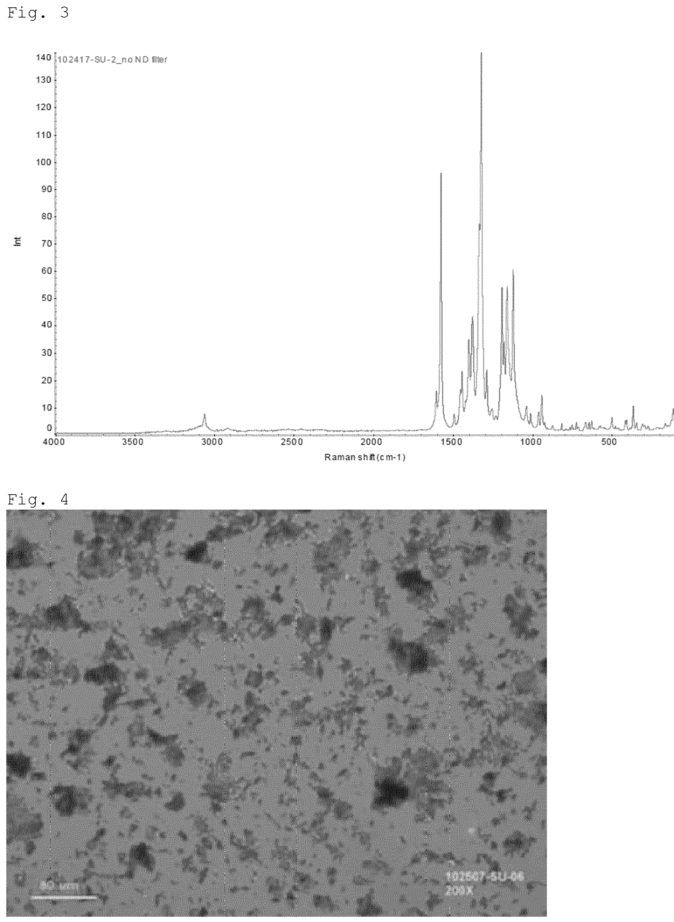

X-ray powder diffraction (Measurement condition 1, see Figure 1) (2θ): 6.9°, 13.3°, 14.2°, 15.0°, 17.1°, 18.5°, 19.6°, 21.1°, 22.9°, 23.7°, 26.7°, 27.4°, 29.5°, 34.1°, 36.1° Differential scanning calorimetry (DSC) and thermogravimetric analysis (TGA): see Figure 2 Raman scattering spectrum (FT-Raman): see Figure 3 Photomicrograph (PLM): see Figure 4

### Preparation of anhydride crystal

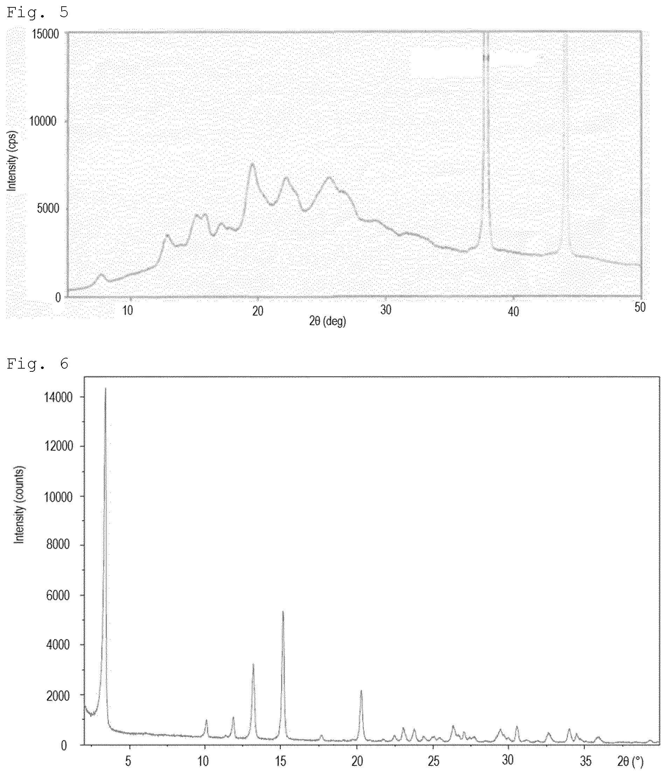

KUS121 (71.8 g) and ethanol (5840 g) were placed in a flask and the mixture was stirred at 76°C to 77°C (reflux) for 30 minutes to be dissolved. Under stirring, the mixture was cooled to 65°C to 70°C and filtered while hot to remove insoluble matters. This operation was repeated in three batches, and the filtrate was combined into one batch. Under stirring, the mixture was cooled to 27°C using a water bath, and further cooled to 4°C using an ice bath. Under stirring, the precipitated solid was aged at 3°C to 4°C for one hour, and the aged crystal was collected on a filter and washed three times with 292 g of ethanol. The crystal was dried under reduced pressure at 30°C to 80°C for 61 hours to obtain a crystal of KUS121 anhydride (yield: 54.8 %).
Water content: 2.3 %_{∘}
X-ray powder diffraction (Measurement condition 3, see Figure 5) (2θ): 7.2°, 13.2°, 15.4°, 16.2°, 17.5°, 20.0°, 22.6°, 25.9°, 27.3°

### Preparation of monohydrate crystal

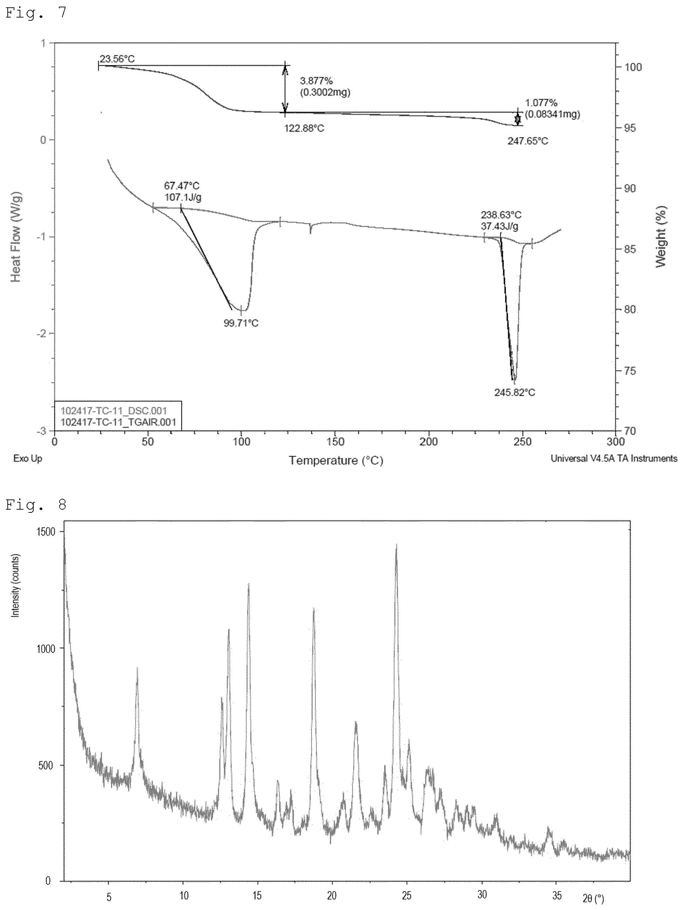

The crystal of KUS121 dihydrate (67 mg) obtained in the above procedure was dried under reduced pressure (17 in Hg, 40°C) while blowing dry nitrogen to obtain a crystal of KUS121 monohydrate (yield: 100 %).
Water content: 3.4 %
X-ray powder diffraction (Analysis condition 2, see Figure 6) (2θ): 10.1°, 10.9°, 13.2°, 15.2°, 20.4°, 23.1°, 23.8°, 26.4°, 29.6°, 30.6°
Differential scanning calorimetry (DSC) and thermogravimetric analysis (TGA): see Figure 7

### Preparation of tetrahydrate crystal

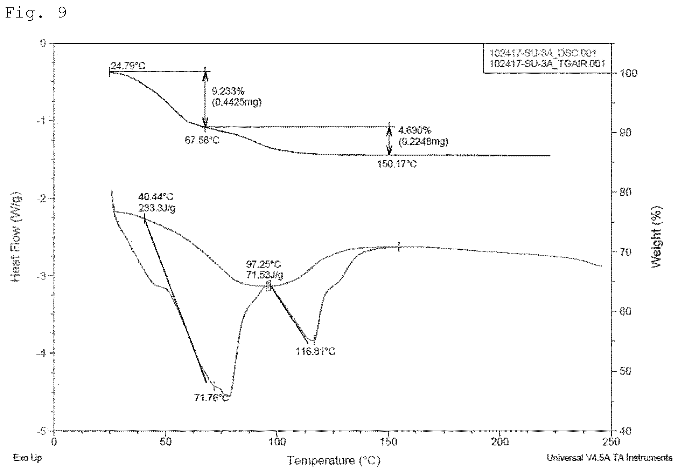

KUS121 (250 mg) was dissolved in 40% ethanol (2.5 mL) at 67°C. The solution was cooled to 60°C at a cooling rate of 0.1°C/min, and stirred for one hour. Further, the solution was cooled to 55°C at a cooling rate of 0.1°C/min, and stirred for one hour to precipitate a crystal. The suspension was cooled to 22°C at a cooling rate of 0.1°C/min, and stirred overnight. The precipitated crystal was filtered by suction and dried with blowing air for one hour to obtain a crystal of KUS121 tetrahydrate (yield: 74 %).
Water content: 13.9 %
X-ray powder diffraction (Analysis condition 2, see Figure 8) (2θ): 6.9°, 12.5°, 13.0°, 14.4°, 16.3°, 18.8°, 21.5°, 23.5°, 24.3°, 25.1°
Differential scanning calorimetry (DSC) and thermogravimetric analysis (TGA): see Figure 9

### (3) Stability test

The crystal of KUS121 dihydrate was subjected to a normal storage stability test (25°C/60% RH, 18 months) and a storage stability test under accelerated test condition (40°C/75% RH, 6 months). For comparison, the crystal of KUS121 anhydride was also subjected to storage stability tests under accelerated test condition (40°C/75% RH, 6 months) and under ambient temperature condition (30 months). The results are shown in tables below.

In addition, the crystal of KUS121 monohydrate and the crystal of KUS121 tetrahydrate were also subjected to storage stability observations. The results are also shown in a table below.

| Crystal of KUS121 dihydrate | | | |
|---|---|---|---|
| Test items | Storage conditions (storage period) | | |
| | Initial | 40°C/75 %RH (6 months) | 25°C/60 %RH (18 months) |
| Related substance | 0.07 % | 0.09 % | 0.11 % |
| Content | 99.7 % | 99.6 % | 99.6 % |
| Water content | 7.4 % | 7.4 % | 7.4 % |
| XRPD | dihydrate crystal | the crystalline form maintained | the crystalline form maintained |

| Crystal of KUS121 anhydride | | | |
|---|---|---|---|
| Test items | Storage conditions (storage period) | | |
| | Initial | 40°C/75 %RH (6 months) | Ambient temperature (30 months) |
| Purity test | 99.5 % | 99.5 % | - |
| Content | 98.2 % | 100.8 % | - |
| Water content | 0.8 % | 3.0 % | 9.1 % |
| XRPD | anhydride crystal | Peak disappearance was observed throughout the measurement range. | A diffraction pattern of the tetrahydrate crystal was observed. |

| Stability of other crystals of KUS121 | |
|---|---|
| Crystalline form | Stability |
| Crystal of KUS121 monohydrate | When the monohydrate crystal was left at room temperature, it gradually transformed to the dihydrate crystal, and the higher the humidity, the faster the transition. |
| Crystal of KUS121 tetrahydrate | Crystal water of the tetrahydrate crystal was released at around 40°C or higher. Furthermore, the crystallinity decreased due to drying under reduced pressure. |

As mentioned above, the dihydrate crystal had better storage stability than other hydrate crystals, and was extremely stable even under accelerated test condition.

## Claims

1. A crystal of sodium 4-amino-3-[6-(4-fluoro-2-methylphenyl)pyridin-3-ylazo]naphthalene-1-sulfonate dihydrate, **characterized by** an X-ray powder diffraction pattern having at least four diffraction angle (2θ° ± 0.5°) peaks selected from 6.9°, 13.3°, 14.2°, 15.0°, 17.1°, 18.5°, 19.6°, 21.1°, 22.9°, 23.7°, 26.7°, 27.4°, 29.5°, 34.1°, and 36.1°.

2. The crystal of claim 1, **characterized by** an X-ray powder diffraction pattern having at least four diffraction angle (2θ° ± 0.2°) peaks selected from 6.9°, 13.3°, 14.2°, 15.0°, 17.1°, 18.5°, 19.6°, 21.1°, 22.9°, 23.7°, 26.7°, 27.4°, 29.5°, 34.1°, and 36.1°.

3. The crystal of claim 1 or 2, wherein the selected diffraction angle peaks are at least five peaks.

4. The crystal of claim 1 or 2, wherein the selected diffraction angle peaks are at least 10 peaks.

5. The crystal of claim 3, wherein the selected diffraction angle peaks are 6.9°, 13.3°, 14.2°, 15.0°, and 17.1°.

6. The crystal of claim 4, wherein the selected diffraction angle peaks are 6.9°, 13.3°, 14.2°, 15.0°, 17.1°, 19.6°, 21.1°, 26.7°, 27.4°, and 29.5°.

7. A process for preparing a crystal of sodium 4-amino-3-[6-(4-fluoro-2-methylphenyl)pyridin-3-ylazo]naphthalene-1-sulfonate dihydrate, comprising
(i) dissolving sodium 4-amino-3-[6-(4-fluoro-2-methylphenyl)pyridin-3-ylazo]naphthalene-1-sulfonate in a water-containing solvent with appropriate heating, said solvent is a solvent commonly used in the manufacture of pharmaceuticals, wherein the solvent commonly used is an alcoholic solvent, and stirring the solution at 40°C to 100°C,
(ii) gradually cooling the solution to precipitate a crystal, and collecting the precipitated crystal on a filter, and
(iii) drying the crystal at a temperature of 30°C to 70°C in an atmosphere with relative humidity of 10% RH to 100% RH to give a crystal of sodium 4-amino-3-[6-(4-fluoro-2-methylphenyl)pyridin-3-ylazo]naphthalene-1-sulfonate dihydrate.

8. The process for preparing a crystal of sodium 4-amino-3-[6-(4-fluoro-2-methylphenyl)pyridin-3-ylazo]naphthalene-1-sulfonate dihydrate according to claim 7, comprising
(i) dissolving sodium 4-amino-3-[6-(4-fluoro-2-methylphenyl)pyridin-3-ylazo]naphthalene-1-sulfonate in a water-containing solvent at a concentration of 1% (w/v) to 20%(w/v) with appropriate heating, said solvent is a solvent commonly used in the manufacture of pharmaceuticals, wherein the solvent commonly used is an alcoholic solvent, and stirring the solution at 40°C to 100°C for 5 min or more,
(ii) cooling the solution to -20°C to 30°C at a cooling rate of 0.01°C/min to 1°C/min, and collecting the precipitated crystal on a filter, and
(iii) drying the crystal at a temperature of 30°C to 70°C in an atmosphere with relative humidity of 10% RH to 100% RH to give a crystal of sodium 4-amino-3-[6-(4-fluoro-2-methylphenyl)pyridin-3-ylazo]naphthalene-1-sulfonate dihydrate.

9. The process of claim 7 or 8, wherein the alcoholic solvent is methanol, ethanol, 1-propanol, and/or 2-propanol.

10. The process of any one of claims 7 to 9, wherein the drying step in (iii) is done under ambient pressure for 10 hours or more.

11. The process of any one of claims 7 to 9, wherein the drying step in (iii) is done at a temperature of 30°C to 50°C and a pressure of 2 kPa to 5 kPa for 10 hours or more in a drying chamber in which a tray filled with water is placed.

12. The process of any one of claims 7 to 9, wherein the drying step in (iii) is done at a temperature of 30°C to 50°C and a pressure of 1 kPa to 7 kPa for 5 hours or more in a drying chamber, while blowing nitrogen saturated with water vapor into the dry chamber.

## Patentansprüche

1. Kristall von Natrium-4-amino-3-[6-(4-fluor-2-methylphenyl)pyridin-3-ylazo]naphthalin-1-sulfonat-Dihydrat, **gekennzeichnet durch** ein Röntgenpulverdiffraktionsmuster mit mindestens vier Beugungswinkelpeaks (2θ° ± 0,5°), ausgewählt aus 6,9°, 13,3°, 14,2°, 15,0°, 17,1°, 18,5°, 19,6°, 21,1°, 22,9°, 23,7°, 26,7°, 27,4°, 29,5°, 34,1° und 36,1°.

2. Kristall gemäß Anspruch 1, **gekennzeichnet durch** ein Röntgenpulverdiffraktionsmuster mit mindestens vier Beugungswinkelpeaks (2θ° ± 0,2°), ausgewählt aus 6,9°, 13,3°, 14,2°, 15,0°, 17,1°, 18,5°, 19,6°, 21,1°, 22,9°, 23,7°, 26,7°, 27,4°, 29,5°, 34,1° und 36,1°.

3. Kristall gemäß Anspruch 1 oder 2, wobei die ausgewählten Beugungswinkelpeaks mindestens fünf Peaks umfassen.

4. Kristall gemäß Anspruch 1 oder 2, wobei die ausgewählten Beugungswinkelpeaks mindestens 10 Peaks umfassen.

5. Kristall gemäß Anspruch 3, wobei die ausgewählten Beugungswinkelpeaks 6,9°, 13,3°, 14,2°, 15,0° und 17,1° sind.

6. Kristall gemäß Anspruch 4, wobei die ausgewählten Beugungswinkelpeaks 6,9°, 13,3°, 14,2°, 15,0°, 17,1°, 19,6°, 21,1°, 26,7", 27,4° und 29,5° sind.

7. Verfahren zur Herstellung eines Kristalls von Natrium-4-amino-3-[6-(4-fluor-2-methylphenyl)pyridin-3-ylazo]naphthalin-1-sulfonat-Dihydrat, umfassend
(i) das Auflösen von Natrium-4-amino-3-[6-(4-fluor-2-methylphenyl)pyridin-3-ylazo]naphthalin-1-sulfonat unter geeigneter Erwärmung in einem wasserhaltigen Lösungsmittel, wobei das Lösungsmittel ein bei der Herstellung von Arzneimitteln üblicherweise verwendetes Lösungsmittel ist, wobei das üblicherweise verwendete Lösungsmittel ein alkoholisches Lösungsmittel ist, und Rühren der Lösung bei 40 °C bis 100 °C,
(ii) die schrittweise Abkühlung der Lösung, um einen Kristall auszufällen, und auffangen des ausgefällten Kristalls auf einem Filter, und
(iii) Trocknen des Kristalls bei einer Temperatur von 30 °C bis 70 °C in einer Atmosphäre mit einer relativen Luftfeuchtigkeit von 10% RH bis 100% RH, um einen Kristall von Natrium-4-amino-3-[6-(4-fluor-2-methylphenyl)pyridin-3-ylazo]naphthalin-1-sulfonat-Dihydrat zu erhalten.

8. Verfahren zur Herstellung eines Kristalls von Natrium-4-amino-3-[6-(4-fluor-2-methylphenyl)pyridin-3-ylazo]naphthalin-1-sulfonat-Dihydrat gemäß Anspruch 7, umfassend
(i) das Auflösen von Natrium-4-amino-3-[6-(4-fluor-2-methylphenyl)pyridin-3-ylazo]naphthalin-1-sulfonat in einem wasserhaltigen Lösungsmittel bei einer Konzentration von 1% (w/v) bis 20% (w/v) unter geeigneter Erwärmung, wobei das Lösungsmittel ein bei der Herstellung von Arzneimitteln üblicherweise verwendetes Lösungsmittel ist, wobei das üblicherweise verwendete Lösungsmittel ein alkoholisches Lösungsmittel ist, und Rühren der Lösung bei 40°C bis 100°C für 5 Minuten oder länger,
(ii) Abkühlen der Lösung auf -20°C bis 30°C mit einer Abkühlgeschwindigkeit von 0,01°C/min bis 1°C/min, und Auffangen des ausgefällten Kristalls auf einem Filter, und
(iii) Trocknen des Kristalls bei einer Temperatur von 30°C bis 70°C in einer Atmosphäre mit einer relativen Luftfeuchtigkeit von 10% RH bis 100% RH, um einen Kristall von Natrium-4-amino-3-[6-(4-fluor-2-methylphenyl)pyridin-3-ylazo]naphthalin-1-sulfonat-Dihydrat zu erhalten.

9. Verfahren gemäß Anspruch 7 oder 8, wobei das alkoholische Lösungsmittel Methanol, Ethanol, 1-Propanol und/oder 2-Propanol ist.

10. Verfahren gemäß mindestens einem der Ansprüche 7 bis 9, wobei der Trocknungsschritt in (iii) unter Umgebungsdruck (ambient pressure) für 10 Stunden oder länger durchgeführt wird.

11. Verfahren gemäß mindestens einem der Ansprüche 7 bis 9, wobei der Trocknungsschritt in (iii) bei einer Temperatur von 30 °C bis 50 °C und einem Druck von 2 kPa bis 5 kPa für 10 Stunden oder länger in einer Trockenkammer durchgeführt wird, in der eine mit Wasser gefüllte Schale angeordnet ist.

12. Verfahren gemäß mindestens einem der Ansprüche 7 bis 9, wobei der Trocknungsschritt in (iii) bei einer Temperatur von 30 °C bis 50 °C und einem Druck von 1 kPa bis 7 kPa für 5 Stunden oder länger in einer Trockenkammer durchgeführt wird, während mit Wasserdampf gesättigter Stickstoff in die Trockenkammer eingeblasen wird.

## Revendications

1. Cristal de dihydrate de 4-amino-3-[6-(4-fluoro-2-méthylphényl)pyridin-3-ylazo]naphtalène-1-sulfonate de sodium, **caractérisé par** un diagramme de diffraction des rayons X sur poudre présentant au moins quatre pics aux angles de diffraction (2θ ± 0,5°) sélectionnés parmi 6,9°, 13,3°, 14,2°, 15,0°, 17,1°, 18,5°, 19,6°, 21,1°, 22,9°, 23,7°, 26,7°, 27,4°, 29,5°, 34,1° et 36,1°.

2. Cristal selon la revendication 1, **caractérisé par le fait qu'**un diagramme de diffraction des rayons X sur poudre a au moins quatre pics aux angles de diffraction (2θ ± 0,2°) sélectionnés parmi 6,9°, 13,3°, 14,2°, 15,0°, 17,1°, 18,5°, 19,6°, 21,1°, 22,9°, 23,7°, 26,7°, 27,4°, 29,5°, 34,1° et 36,1°.

3. Cristal selon la revendication 1 ou la revendication 2, dans lequel les pics aux angles de diffraction sélectionnés sont au moins cinq pics.

4. Cristal selon la revendication 1 ou la revendication 2, dans lequel les pics aux angles de diffraction sélectionnés sont au moins 10 pics.

5. Cristal selon la revendication 3, dans lequel les pics aux angles de diffraction sélectionnés sont 6,9°, 13,3°, 14,2°, 15,0° et 17,1°.

6. Cristal selon la revendication 4, dans lequel les pics aux angles de diffraction sélectionnés sont 6,9°, 13,3°, 14,2°, 15,0°, 17,1°, 19,6°, 21,1°, 26,7°, 27,4° et 29,5°.

7. Procédé de préparation d'un cristal de 4-amino-3-[6-(4-fluoro-2-méthylphényl)pyridin-3-ylazo]naphtalène-1-sulfonate de sodium dihydraté, comprenant
(i) le fait de dissoudre 4-amino-3-[6-(4-fluoro-2-méthylphényl)pyridin-3-ylazo]naphtalène-1-sulfonate de sodium dans un solvant contenant de l'eau avec un chauffage approprié, ledit solvant étant un solvant couramment utilisé dans la fabrication de produits pharmaceutiques, le solvant couramment utilisé étant un solvant alcoolique, et le fait d'agiter la solution à une température allant de 40 °C à 100 °C,
(ii) le fait de refroidir progressivement la solution de manière à précipiter un cristal et de collecter le cristal précipité sur un filtre, et
(iii) le fait de sécher le cristal à une température allant de 30 °C à 70 °C dans une atmosphère présentant une humidité relative allant de 10 % RH à 100 % RH pour donner un cristal de dihydrate de 4-amino-3-[6-(4-fluoro-2-méthylphényl)pyridin-3-ylazo]naphtalène-1-sulfonate de sodium.

8. Procédé pour préparer un cristal de dihydrate de 4-amino-3-[6-(4-fluoro-2-méthylphényl)pyridin-3-ylazo]naphtalène-1-sulfonate de sodium selon la revendication 7, comprenant
(i) le fait de dissoudre du 4-amino-3-[6-(4-fluoro-2-méthylphényl)pyridin-3-ylazo]naphtalène-1-sulfonate de sodium dans un solvant contenant de l'eau à une concentration allant de 1 % (p/v) à 20 % (p/v) avec un chauffage approprié, ledit solvant étant un solvant couramment utilisé dans la fabrication de produits pharmaceutiques, le solvant couramment utilisé étant un solvant alcoolique, et le fait d'agiter la solution à 40 °C à 100 °C pendant 5 min ou plus,
(ii) le fait de refroidir la solution jusqu'à -20 °C à 30 °C à une vitesse de refroidissement allant de 0,01 °C/min à 1 °C/min et le fait de collecter le cristal précipité sur un filtre, et
(iii) le fait de sécher le cristal à une température allant de 30 °C à 70 °C dans une atmosphère présentant une humidité relative de 10 % RH à 100 % RH pour donner un cristal de dihydrate de 4-amino-3-[6-(4-fluoro-2-méthylphényl)pyridin-3-ylazo]naphtalène-1-sulfonate de sodium.

9. Procédé selon la revendication 7 ou la revendication 8, dans lequel le solvant alcoolique est du méthanol, de l'éthanol, du 1-propanol et/ou du 2-propanol.

10. Procédé selon l'une quelconque des revendications 7 à 9, dans lequel l'étape de séchage en (iii) est effectuée sous pression ambiante pendant 10 heures ou plus.

11. Procédé selon l'une quelconque des revendications 7 à 9, dans lequel l'étape de séchage en (iii) est effectuée à une température de 30 °C à 50 °C et à une pression de 2 kPa à 5 kPa pendant 10 heures ou plus dans une chambre de séchage dans laquelle un plateau rempli d'eau est placé.

12. Procédé selon l'une quelconque des revendications 7 à 9, dans lequel l'étape de séchage en (iii) est effectuée à une température allant de 30 °C à 50 °C et à une pression allant de 1 kPa à 7 kPa pendant 5 heures ou plus dans une chambre de séchage, tout en soufflant dans la chambre de séchage de l'azote saturé avec de la vapeur d'eau.
